# EUROPEAN PATENT APPLICATION

(11) **EP 4 751 691 A1**
(43) Date of publication of application: **03.06.2026**
(21) Application number: 24912255.7
(22) Date of filing: 04.12.2024
(51) Int. Cl.: A61F 13/15, A61F 13/56, A61F 13/551

(54) **ABSORBENT ARTICLE**

(30) Priority: 27.12.2023 JP 2023221896
(71) Applicant: Unicharm Corporation, Ehime 799-0111 (JP)
(72) Inventor: SHIMBO, Yosuke, Kanonji-shi, Kagawa 769-1602 (JP); SHIMAZU, Takeshi, Kanonji-shi, Kagawa 769-1602 (JP); NASHIKI, Kento, Kanonji-shi, Kagawa 769-1602 (JP)
(74) Representative: Staeger & Sperling Partnerschaftsgesellschaft mbB
(86) International application number: PCT/JP2024/042885
(87) International publication number: WO 2025/142365

(57) **Abstract**

An absorbent article (1) comprises a liquid-absorbing absorbent core (10) and has a longitudinal direction, a width direction, and a thickness direction, these directions being orthogonal to each other while the absorbent article (1) is in an unfolded state, the absorbent article (1) being characterized in having self-adhesive joining parts (30), the joining parts (30) being provided on one side of a folding line along which the absorbent article (1) is folded when packaged, and on the other side of the folding line, with respect to a direction orthogonal to the folding line.

## Description

### FIELD

The present invention relates to an absorbent article.

### BACKGROUND

Patent Document 1 discloses, as an absorbent article, diaper pants including a chassis and a ring-like elastic belt. The document discloses that the seams (side portions) between the front and rear waist regions of the diaper pants are tucked into the chassis before packaging.

### [CITATION LIST]

### [PATENT LITERATURE]

[Patent Document 1] Japanese Patent No. 6013616

### SUMMARY

### [TECHNICAL PROBLEM]

Compact packaging can be achieved by folding the side portions of the waist regions before packaging, as described above. However, in Patent Document 1, the side portions of the waist regions are merely tucked in, and this state is difficult to maintain. Thus, for example, when discarding the diaper pants, even if the user tucks the side portions of the waist regions into the chassis like when the product was packaged, the folds of the waist regions cannot be maintained and will open up, making it difficult to discard the product in a compact state.

Such folds could be maintained, for example, by providing an adhesive on the absorbent article. However, simply providing an adhesive may cause the added adhesive to stick to the wearer's skin or clothing when the absorbent article is worn, which may make it difficult to attach the absorbent article in a proper position.

The present invention was achieved in light of problems such as those described above, and an objective thereof is to provide an absorbent article capable of maintaining a compact state while ensuring easy wearability.

### [SOLUTION TO PROBLEM]

A main aspect of the invention for achieving the above-described objective is an absorbent article having, in an unfolded state, a lengthwise direction, a width direction, and a thickness direction orthogonal to one another, the absorbent article including: a liquid-absorbent absorbent core; and a self-adhesive joining portion, the joining portion being disposed, in a direction orthogonal to a fold line upon packaging, on one side relative to the fold line and also on an other side relative to the fold line.

Other features of the present invention will become clear through the description of the present specification with reference to the accompanying drawings.

### [ADVANTAGEOUS EFFECT OF THE INVENTION]

According to the present invention, it is possible to provide an absorbent article capable of maintaining a compact state while ensuring easy wearability.

### BRIEF DESCRIPTION OF DRAWINGS

FIG. 1 is a schematic plan view of urine collection pad 1 in an unfolded and stretched state as viewed from the skin side.
FIG. 2 is a schematic cross-sectional view of urine collection pad 1.
FIG. 3 includes diagrams illustrating pad 1 when packaged.
FIG. 4 is a diagram illustrating self-adhesive joining portions 30.
FIG. 5 is a diagram illustrating a modified example of joining portions 30.
FIG. 6 is a diagram illustrating a modified example of joining portions 30.
FIG. 7 is a diagram illustrating a modified example of joining portions 30.
FIG. 8 is a diagram illustrating a modified example of joining portions 30.
FIG. 9 includes diagrams illustrating configurations of joining portions 30.
FIG. 10 includes plan views of joining portions 30.
FIG. 11 includes diagrams illustrating three-fold-type pads 1.
FIG. 12 includes diagrams illustrating self-adhesive joining portions 50 of diaper 40.
FIG. 13 includes diagrams illustrating self-adhesive joining portions 50 of diaper 40.

### DESCRIPTION OF EMBODIMENTS

At least the following matters are disclosed in the description of this specification and the attached drawings.

### Aspect 1:

An absorbent article having, in an unfolded state, a lengthwise direction, a width direction, and a thickness direction orthogonal to one another, the absorbent article including: a liquid-absorbent absorbent core; and a self-adhesive joining portion, the joining portion being disposed, in a direction orthogonal to a fold line upon packaging, on one side relative to the fold line and also on an other side relative to the fold line.

According to Aspect 1, by bringing the one-side joining portion and the other-side joining portion into contact while folding the absorbent article in the orthogonal direction, adhesive force is exerted, and the absorbent article is maintained in a compact folded state. Further, by employing self-adhesive joining portions, when the absorbent article is worn, the joining portions do not adhere to the wearer or to items such as clothing, thereby facilitating alignment of the absorbent article relative to the wearer and also ensuring easy wearability.

### Aspect 2:

The absorbent article according to Aspect 1, wherein when the absorbent article is folded at a center line of the absorbent article in the lengthwise direction, the joining portion includes a two-layer portion superposed in the thickness direction.

According to Aspect 2, when the absorbent article is folded in two in the lengthwise direction, the one-side joining portion and the other-side joining portion contact each other to exhibit adhesive force. Thus, the absorbent article can be maintained in a compact, two-folded state in the lengthwise direction.

### Aspect 3:

The absorbent article according to Aspect 2, wherein the joining portion includes a first joining portion disposed on the one side relative to the fold line and a second joining portion disposed on the other side relative to the fold line, and when the absorbent article is divided in four in the lengthwise direction, the first joining portion is disposed in an end region on one side, and the second joining portion is disposed in an end region on an other side.

According to Aspect 3, it is possible to reduce parts of the absorbent article that would otherwise open up outside the joining portions in the lengthwise direction, thereby easily maintaining the absorbent article in a compact folded state.

### Aspect 4:

The absorbent article according to Aspect 3, wherein the joining portion includes a first joining portion disposed on the one side relative to the fold line and a second joining portion disposed on the other side relative to the fold line, and when the absorbent article is divided in four in the lengthwise direction, the first joining portion is disposed in a first central region adjacent to an end region on one side, and the second joining portion is disposed in a second central region adjacent to an end region on an other side.

According to Aspect 4, the joining portions are located close to the fold position, and thus, it is easier for the user to align the one-side joining portion and the other-side joining portion and to bring the joining portions into contact with each other.

### Aspect 5:

The absorbent article according to any one of Aspects 1 to 4, wherein the joining portion is disposed on a skin-side surface of the absorbent article.

According to Aspect 5, the folded state of the absorbent article can be maintained with its skin-side surface-which is for receiving and absorbing excreted fluid-facing inward, thereby enabling hygienic discarding of the absorbent article. Further, excreted fluid and odor therefrom can be contained inside the folded absorbent article.

### Aspect 6:

The absorbent article according to any one of Aspects 1 to 5, wherein a length of the joining portion in the lengthwise direction is longer than a length of the joining portion in the width direction.

According to Aspect 6, a wide region of the absorbent article in the lengthwise direction can be joined together by the joining portions, thereby making it easy to maintain the absorbent article in a compact folded state.

### Aspect 7:

The absorbent article according to any one of Aspects 1 to 6, wherein the joining portion is disposed on a non-hydrophilic sheet.

According to Aspect 7, since a non-hydrophilic sheet is less likely to absorb and retain excreted fluid, the joining portions are less likely to contact excreted fluid, thereby inhibiting deterioration in adhesive force between the joining portions.

### Aspect 8:

The absorbent article according to any one of Aspects 1 to 6, wherein the joining portion is disposed on a hydrophilic sheet.

According to Aspect 8, since excreted fluid diffuses on a hydrophilic sheet and pooling of fluid is thus less likely to occur, it is possible to inhibit deterioration in adhesive force between the joining portions caused by pooling of fluid.

### Aspect 9:

The absorbent article according to any one of Aspects 1 to 8, wherein the joining portion exhibits adhesive force by a material other than a hook-and-loop fastener and an adhesive.

According to Aspect 9, when the absorbent article is worn, the joining portions do not adhere to the wearer or to items such as clothing, thereby facilitating alignment of the absorbent article relative to the wearer and also ensuring easy wearability.

### Aspect 10:

The absorbent article according to Aspect 5, wherein a mean deviation of surface roughness on a joining face of the joining portion is 20 or less.

According to Aspect 10, the texture of the joining portion against the skin can be improved and an unnatural feeling during wear can be alleviated, compared to cases where the mean deviation of surface roughness is greater than 20.

### Aspect 11:

The absorbent article according to any one of Aspects 1 to 10, wherein a mean frictional coefficient on a joining face of the joining portion is 0.2 or greater.

According to Aspect 11, slipperiness of the joining portion can be reduced, compared to cases where the mean frictional coefficient is less than 0.2. Thus, the absorbent article is less likely to be misaligned with respect to the wearer or the outer garment, thereby alleviating an unnatural feeling during wear.

### Aspect 12:

The absorbent article according to any one of Aspects 1 to 11, wherein when an adhesive force measurement test is performed to measure an adhesive force between the joining portion after repeatedly joining and peeling the joining portion, the adhesive force between the joining portion is 0.2 N/25 mm or greater.

According to Aspect 12, adhesive force can be maintained even after repeatedly joining and peeling the joining portions, compared to cases where the adhesive force is less than 0.2 N/25 mm. Therefore, even when a user repeatedly joins and peels the joining portions until the one-side joining portion and the other-side joining portion are brought into contact appropriately, the absorbent article can be maintained in its compact folded state.

### Aspect 13:

The absorbent article according to any one of Aspects 1 to 12, wherein the joining portion includes a first joining portion disposed on the one side relative to the fold line and a second joining portion disposed on the other side relative to the fold line, and an area of the first joining portion is different from an area of the second joining portion.

According to Aspect 13, the user can easily align the smaller joining portion with respect to the larger joining portion and easily bring the joining portions into contact with each other.

### Aspect 14:

The absorbent article according to any one of Aspects 1 to 13, wherein the absorbent article comprises a presence region in which the absorbent core is present, and an absence region in which the absorbent core is not present, and the joining portion is disposed in the presence region.

According to Aspect 14, by disposing the joining portions in the presence region having high stiffness, the force applied by the user to bring the joining portions into contact can be readily transmitted, and the joining portions can be joined together stably and securely.

### Aspect 15:

The absorbent article according to any one of Aspects 1 to 13, wherein the absorbent article comprises a presence region in which the absorbent core is present, and an absence region in which the absorbent core is not present, and the joining portion is disposed in the absence region.

According to Aspect 15, the joining portions can be disposed in the peripheral edge portion of the absorbent article, thereby making it easy to maintain the absorbent article in a folded state. Further, it is possible to reduce parts of the absorbent article that would otherwise open up outside the joining portions.

### Aspect 16:

The absorbent article according to any one of Aspects 1 to 13, wherein the absorbent article comprises a presence region in which the absorbent core is present, and an absence region in which the absorbent core is not present, and the joining portion is disposed extending across the presence region and the absence region.

According to Aspect 16, the joining portions can be joined together stably and securely by a part of the joining portions disposed in the highly-stiff presence region, while also disposing the joining portions in the peripheral edge portion of the absorbent article.

### Aspect 17:

The absorbent article according to any one of Aspects 1 to 13, wherein the joining portion includes a first joining portion disposed on the one side relative to the fold line and a second joining portion disposed on the other side relative to the fold line, the absorbent article comprises a presence region in which the absorbent core is present, and an absence region in which the absorbent core is not present, and one of the first joining portion and the second joining portion is disposed in the presence region, and the other is disposed in the absence region.

According to Aspect 17, the other joining portion can be joined stably and securely to the one joining portion disposed in the highly-stiff presence region, while disposing the other joining portion in the peripheral edge portion of the absorbent article.

### Aspect 18:

The absorbent article according to any one of Aspects 1 to 17, wherein the joining portion is disposed in a widthwise central region when the absorbent article is divided in three in the width direction.

According to Aspect 18, opening of both lateral sides of the absorbent article, which are located further outward in the width direction than the joining portions, can be inhibited in a balanced manner.

### Aspect 19:

The absorbent article according to any one of Aspects 1 to 17, wherein the joining portion is disposed in a widthwise end region when the absorbent article is divided in three in the width direction.

According to Aspect 19, the user can bring the one-side joining portion and the other-side joining portion into contact while aligning the end portions of the absorbent article.

### Aspect 20:

The absorbent article according to any one of Aspects 1 to 19, wherein the joining portion includes a plurality of sets of a first joining portion disposed on the one side relative to the fold line and a second joining portion disposed on the other side relative to the fold line.

According to Aspect 20, by including a plurality of sets of intermittently-disposed joining portions, it is possible to inhibit the joining portions from increasing the stiffness of the absorbent article, while maintaining the absorbent article in a compact folded state.

### FIRST EMBODIMENT:

In a first embodiment, a urine collection pad will be described as an example of an absorbent article according to the present invention. Note, however, that the absorbent article according to the present invention is not limited to urine collection pads, and is applicable to, for example, sanitary napkins, underpants-shaped disposable diapers, panty-shaped napkins, tape-type disposable diapers, diapers for pet use, absorbent sheets for caregiving or for pet use, and the like.

### Basic Configuration of Urine Collection Pad 1:

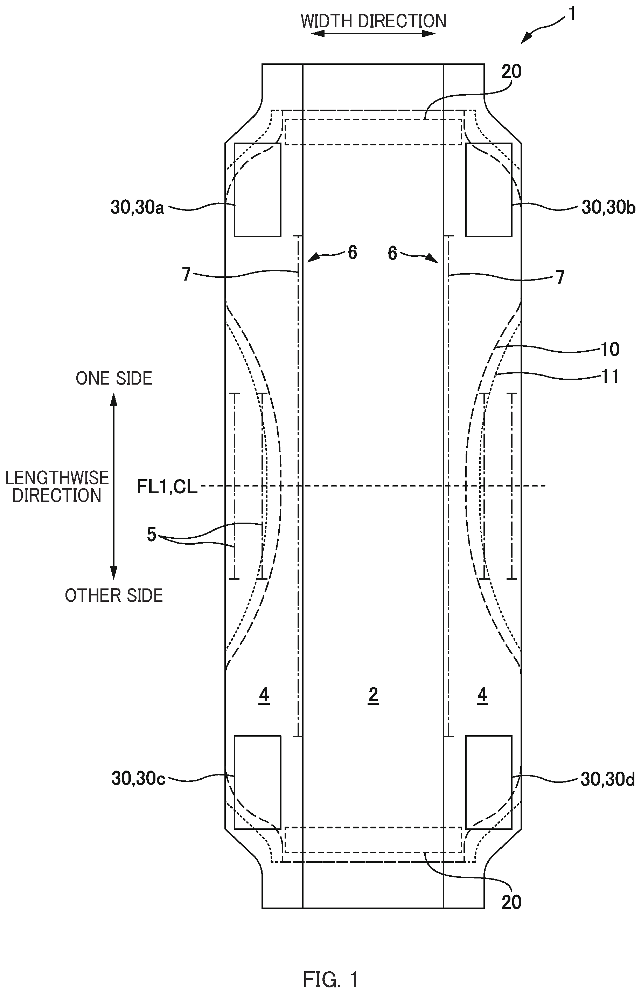

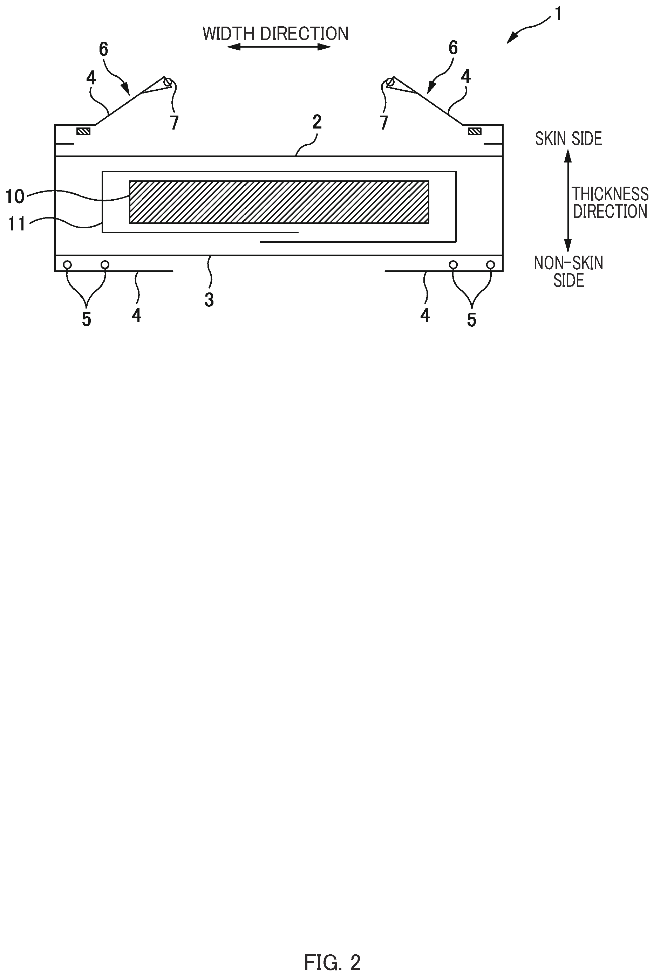

FIG. 1 is a schematic plan view of a urine collection pad 1 in an unfolded and stretched state as viewed from the skin side. FIG. 2 is a schematic cross-sectional view of the urine collection pad 1. The urine collection pad 1 (also referred to hereinafter as "pad") can be used by being arranged on the inner side of an underpants-shaped or tape-type disposable diaper, or the pad 1 alone can be used by being directly arranged on the inner side of the wearer's underwear (underpants or panties).

The pad 1 has a substantially rectangular planar-view shape in an unfolded state, and has a lengthwise direction, a width direction, and a thickness direction orthogonal to one another. The lengthwise direction is along the front-back direction extending from the wearer's front side toward the back side when the pad 1 is worn. In the thickness direction, the side that contacts the wearer's skin is the skin side, whereas the opposite side therefrom is the non-skin side. The pad 1 of the present embodiment is symmetrical in the lengthwise direction, and thus, either side of the pad 1 in the lengthwise direction may correspond to the wearer's front side. Unlike a tape-type or underpants-shaped diaper, the pad 1 is worn in a state where its one-side end portion and the other-side end portion in the lengthwise direction are not connected around the wearer's waist.

The "unfolded state" of the pad 1 refers to a state in which the entire pad 1 is unfolded and spread out in a planar manner. The "stretched state" of the pad 1 refers to a state in which the pad 1 has been stretched to an extent that the wrinkles formed in the pad are substantially visually unrecognizable, and refers to a state in which the pad 1 has been stretched out to an extent that the size of each member (e.g., side sheet 4 described below) constituting the pad 1 matches, or is close to, the size of that member alone.

The pad 1 includes: a liquid-absorbent absorbent core 10; a liquid-permeable top-surface sheet 2 (e.g., a nonwoven fabric) disposed further toward the skin side than the absorbent core 10; a liquid-impermeable back-surface sheet 3 (e.g., a resin film made from polyethylene, polypropylene, or the like) disposed further toward the non-skin side than the absorbent core 10; and a pair of side sheets 4 disposed on both side portions of the pad 1 in the width direction. As illustrated in FIG. 2, each of the side sheets 4 is provided from the side portion, in the width direction, of the non-skin-side surface of the back-surface sheet 3 and is folded inward in the width direction toward the skin-side surface of the skin-side sheet 2 so as to wrap the absorbent core 10.

An example of the absorbent core 10 may be a member wherein a liquid-absorbent fiber, such as pulp fiber, containing superabsorbent polymer (SAP) is formed into a predetermined shape. The absorbent core 10 is covered by a liquid-permeable core-wrapping sheet 11 (e.g., tissue paper). However, the above is not a limitation, and other examples of the absorbent core 10 may include a SAP sheet made by attaching a SAP layer on a hydrophilic sheet or an air-laid sheet made by forming liquid-absorbent fiber into a sheet shape by air-laying, or the absorbent core may have a layered structure including two or more layers. Further, the absorbent core 10 does not have to be covered by a core-wrapping sheet 11.

The pad 1 has, on its non-skin-side surface, fixing members 20 for joining the pad 1 to the inner surface of an underpants-shaped diaper or underwear (outer garment). The fixing members 20 are provided respectively on both end portions of the pad 1 in the lengthwise direction. The fixing member 20 is a rectangular member and is provided such that its long side is oriented along the width direction of the pad 1. It is preferable that the fixing member 20 is a member that is peelable from the inner surface of the outer garment without damaging the outer garment, and that is re-attachable. The fixing member 20 of the present embodiment is a tape having a hook member (e.g., a male member of a hook-and-loop fastener). Another example of the fixing member 20 may be a tape coated with an adhesive.

Further, the pad 1 includes, at both side portions in the width direction, a pair of leg elastic members 5 provided along the lengthwise direction. In FIG. 2, the leg elastic members 5 are fixed between the back-surface sheet 3 and the side sheet 4 in a state stretched in the lengthwise direction. With this configuration, both side portions of the pad 1 in the width direction readily fit tightly around the wearer's legs.

Further, the pad 1 includes, at both side portions in the width direction, a pair of leak-proof wall portions 6 capable of standing up toward the skin side. Each leak-proof wall portion 6 is formed by the side sheet 4 and a leak-proof-wall elastic member 7. The leak-proof-wall elastic member 7 is fixed at an inner end portion of the side sheet 4 in the width direction in a state stretched along the lengthwise direction of the pad 1.

A basic configuration of the pad 1 has been described above, but the above configuration of the pad 1 is merely an example and is not limited to the above. For example, the planar-view shape of the pad 1 may be asymmetrical in the lengthwise direction, and/or the pad 1 does not have to include the fixing members 20.

### Self-Adhesive Joining portion 30:

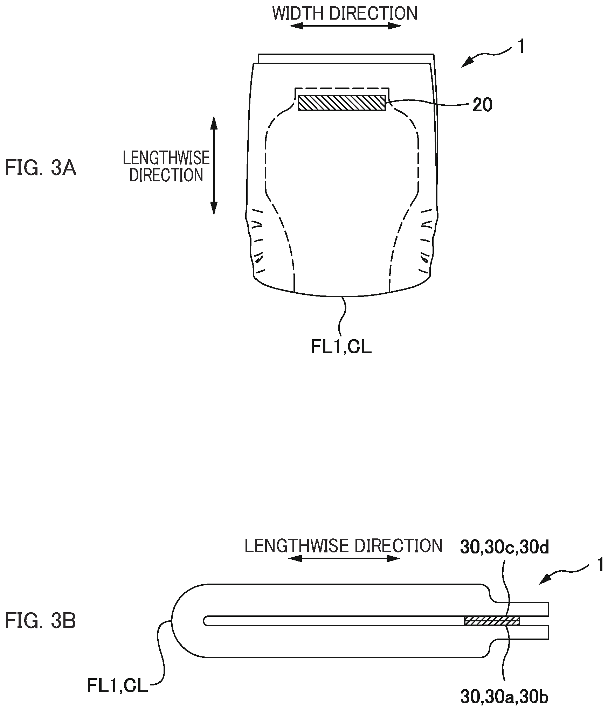

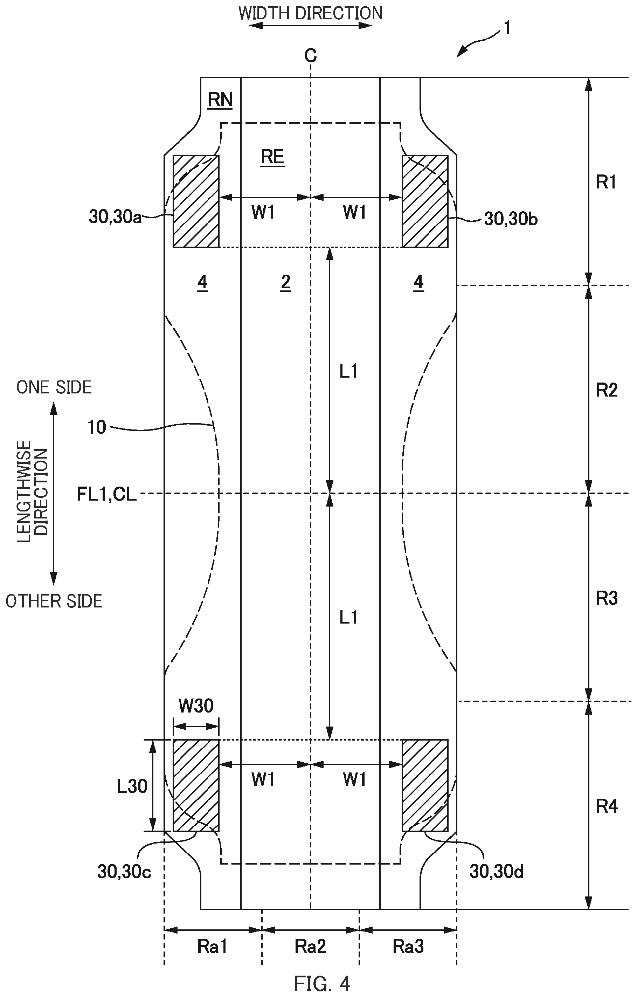

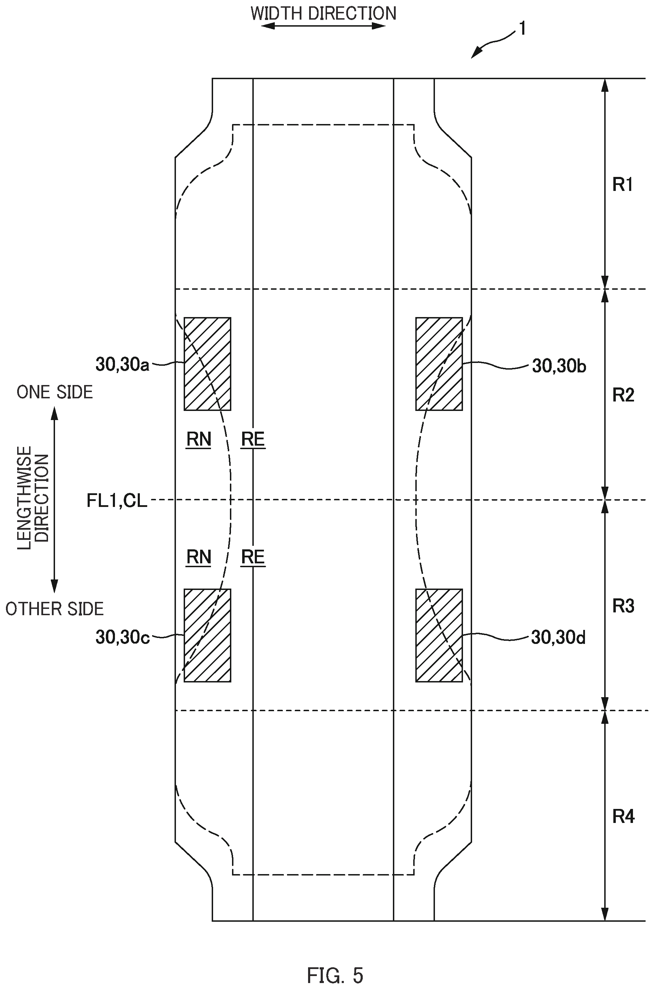

FIG. 3 includes diagrams illustrating the pad 1 when packaged. FIG. 3A is a plan view of the pad 1 when packaged, and FIG. 3B is a schematic cross-sectional view of the pad 1 when packaged. FIG. 4 is a diagram illustrating self-adhesive joining portions 30. FIGS. 5 to 8 are diagrams illustrating modified examples of joining portions 30. FIGS. 4 to 8 are schematic plan views of pads 1 viewed from the skin side.

In the description below, the center line of the pad 1 in the lengthwise direction-i.e., the center line which extends along the width direction and which divides the pad 1 equally in two in the lengthwise direction-is also referred to as "lengthwise center line CL". The center line of the pad 1 in the width direction-i.e., the center line which extends along the lengthwise direction and which divides the pad 1 equally in two in the width direction-is also referred to as "widthwise center line C".

The pad 1 is folded at a fold line FL1 upon packaging and made compact, and in this state, one or a plurality of pads is/are housed in a packaging bag of a package to be shipped. The pad 1 of the present embodiment is folded in two at the lengthwise center line CL, which serves as the fold line FL1 upon packaging, and is housed in a packaging bag in this state (FIG. 3A). Thus, the pad 1 is packaged in a compact state in which the length in the lengthwise direction is reduced.

Further, the pad 1 includes self-adhesive joining portions 30. In the lengthwise direction which is orthogonal to the fold line FL1 upon packaging, the joining portion 30 is disposed on one side relative to the fold line FL1 and also on the other side relative to the fold line FL1.

In the pad 1 illustrated in FIG. 4, the joining portions 30 are disposed on the skin-side surface, and the joining portions 30 include: two joining portions 30a, 30b (first joining portions) disposed on the one side in the lengthwise direction relative to the fold line FL1; and two joining portions 30c, 30d (second joining portions) disposed on the other side in the lengthwise direction relative to the fold line FL1. That is, the pad 1 includes four joining portions 30a to 30d, and these joining portions 30 are each disposed respectively at both side portions in the width direction at both end portions in the lengthwise direction. Each joining portion 30a to 30d has a rectangular shape. In the description below, the joining portion 30 disposed on one side relative to the fold line FL1 is also referred to as "one-side joining portion", and the joining portion 30 disposed on the other side relative to the fold line FL1 is also referred to as "other-side joining portion".

The self-adhesive joining portion 30 has properties such that it has low tack (adhesion) by itself, but the joining portions 30 exhibit adhesive force (tackiness) when brought into contact with each other even under relatively weak pressure, while resisting adhesion to regions other than the joining portion 30 even when a strong pressure is applied. More specifically, it is preferable that the adhesive force between the joining portion 30 and a region other than the joining portion 30 is less than or equal to one-fourth of the adhesive force between the joining portions 30. Further, the joining portions 30 are peelable from each other without damaging the pad 1 and are re-attachable. The joining portion 30 is formed by a self-adhesive joining agent 32.

The self-adhesive joining agent 32 is not particularly limited as long as it is safe even when brought into contact with the wearer's skin, and examples thereof may include natural rubber, synthetic rubber, acrylic resin, polyurethane resin, silicone rubber, and the like. One type may be used alone, or a plurality of types may be used in combination.

According to the aforementioned pad 1, a user (e.g., wearer or caregiver) can fold the pad 1 in the lengthwise direction and bring the joining portions 30a, 30b on one side relative to the fold line FL1 into contact with the joining portions 30c, 30d on the other side, to cause the joining portions 30 to exhibit adhesive force. Thus, the fold of the pad 1 is less likely to open, and the pad 1 can be maintained in its folded state. Particularly, the fold line FL1 upon packaging is a fold line for making the planar size of the pad 1 small, as illustrated in FIG. 3A. Therefore, by providing the joining portions 30 on both sides of the fold line FL1, the user can fold the pad 1 in a direction (in this example, in the lengthwise direction) orthogonal to the fold line FL1 upon packaging, to make the pad compact. Thus, the user can carry or discard the pad 1 which is maintained in a compact state.

Further, the joining portions 30 are self-adhesive. Thus, when the pad 1 is worn, the joining portions 30 do not adhere to the wearer's skin or to clothing or surfaces of the pad 1 other than the joining portions 30, thereby facilitating alignment of the pad 1 relative to the wearer and also ensuring easy wearability of the pad 1. Furthermore, since the joining portions 30 on the skin-side surface of the pad 1 do not adhere to the wearer's skin, the joining portions 30 can be inhibited from causing an unnatural feeling during wear.

It should be noted that the fold line upon packaging is not limited to the fold line FL1 along the width direction, and may be, for example, a fold line along the lengthwise direction. In this case, it is preferable that the joining portions 30 are provided on one side and the other side relative to the fold line in the width direction which is orthogonal to the fold line. With this configuration, the pad 1 can be maintained in a state compactly folded in the width direction.

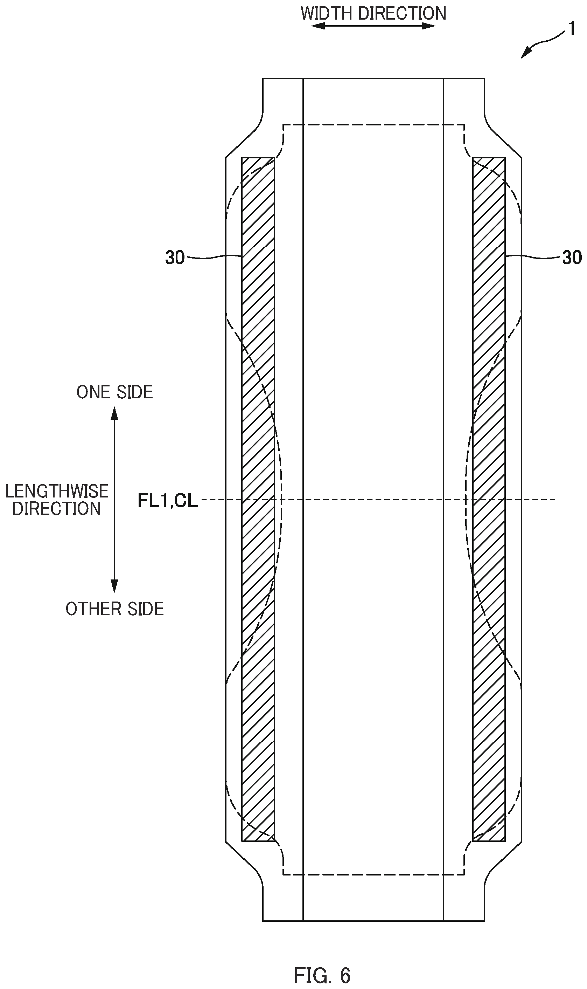

Further, the joining portion 30 on one side relative to the fold line FL1 and the joining portion 30 on the other side relative to the fold line FL1 may be a pair of joining portions 30 separated from each other in the lengthwise direction as illustrated in FIG. 4, or may be continuous in the lengthwise direction as in a modified example illustrated in FIG. 6. In the case of FIG. 6, a portion of the joining portion 30 on one side relative to the fold line FL1 and a portion of the joining portion 30 on the other side come into contact to exhibit adhesive force. In the case of FIG. 6, since the joining portion 30 is present over a wide area of the pad 1 in the lengthwise direction, the fold of the pad 1 is even less likely to open, thereby maintaining a compact state.

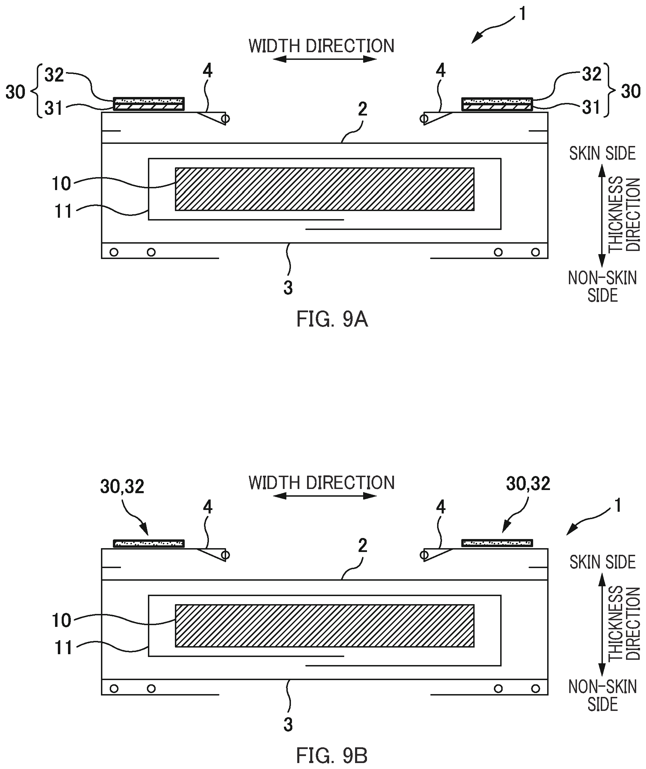

FIGS. 9A and 9B are diagrams illustrating configurations of the joining portions 30, and are schematic cross-sectional views of the pad 1. As illustrated in FIG. 9A, the joining portion 30 is preferably configured by applying the self-adhesive joining agent 32 on a sheet-like base material 31. Alternatively, as illustrated in FIG. 9B, the joining portion 30 may be configured by directly applying the self-adhesive joining agent 32 to a fundamental material (e.g., side sheet 4, top-surface sheet 2, or back-surface sheet 3) constituting the outer surface of the pad 1.

As illustrated in FIG. 9A, by forming the joining portions 30 separately from the fundamental material of the pad 1 and joining them to the fundamental material of the pad 1, the joining portions 30 become more conspicuous. Thus, the user can readily recognize the joining portions 30, which increases the user's awareness of bringing the joining portions 30 together when folding the pad 1. The base material 31 for the separately-formed joining portion 30 is not particularly limited as long as it is a soft sheet-like member, and examples thereof may include nonwoven fabric, resin film, paper, woven fabric, knitted fabric, and the like. Making the base material 31 from a soft sheet can reduce the stiffness of the joining portion 30, which can alleviate an unnatural feeling during wear, even when the joining portions 30 are disposed on the skin-side surface of the pad 1. On the other hand, in the case of FIG. 9B, since the base material 31 is absent, the stiffness of the skin-side surface of the pad 1 can be reduced, thereby further alleviating an unnatural feeling during wear and reducing the number of materials used for the pad 1.

It is preferable that the thickness of the joining portion 30 is 3 mm or less. In this way, the difference in height at the joining portion 30 can be reduced, and an unnatural feeling during wear can be alleviated, compared to cases where the thickness of the joining portion 30 is greater than 3 mm. It should be noted that the thickness can be measured in a non-contact manner using, for example, a laser displacement meter (High-Accuracy 2D Laser Displacement Sensor LJ-G Series (model: LJ-G030) from Keyence Corporation, or an equivalent thereof).

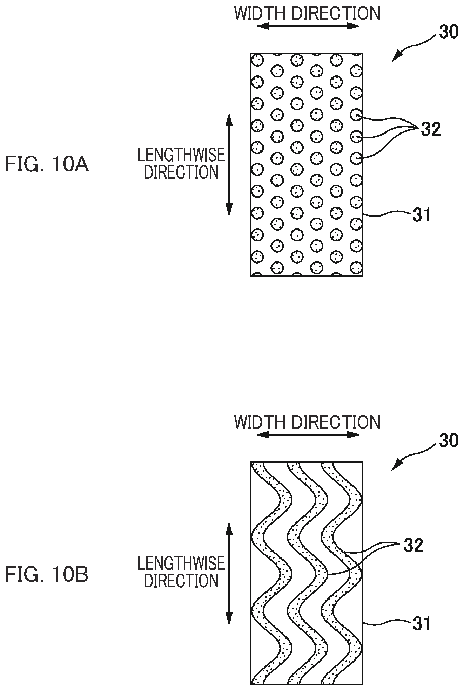

FIGS. 10A and 10B are plan views of the joining portion 30. In the joining portion 30, the self-adhesive joining agent 32 may be applied over the entire region (by so-called solid fill), or the self-adhesive joining agent 32 may be applied according to a predetermined pattern. Examples of predetermined patterns may include a dotted pattern as illustrated in FIG. 10A, a wavy pattern as illustrated in FIG. 10B, and other patterns, such as an Ω-shaped pattern, a spiral pattern, a stripe pattern, or a lattice pattern. The shape of the pattern is not particularly limited. In this case, the joining portion 30 is defined as a region surrounded by imaginary lines that extend in the lengthwise direction and respectively pass through the edge of the self-adhesive joining agent 32 located furthest toward the one side in the width direction and the edge thereof located furthest toward the other side, and imaginary lines that extend in the width direction and respectively pass through the edge of the self-adhesive joining agent 32 located furthest toward the one side in the lengthwise direction and the edge thereof located furthest toward the other side, among the group of elements formed by the self-adhesive joining agent 32 applied according to a predetermined pattern. FIG. 4 illustrates a rectangular joining portion 30, but the shape of the joining portion 30 is not particularly limited.

As described above, the joining portion 30 of the present embodiment exhibits adhesive force 30 by the self-adhesive joining agent 32, such as rubber or the like. That is, the joining portion 30 exhibits adhesive force by a material other than a hook-and-loop fastener and an adhesive. Examples of the hook-and-loop fastener may include a fastener composed of a male member including a plurality of hook-shaped protrusions and a female member including a plurality of loops with which the protrusions on the male member engage, or a fastener composed solely of a male member. That is, the joining portion 30 is different from the fixing member 20 (male member of a hook-and-loop fastener) disposed on the non-skin-side surface of the pad 1 for joining with an outer garment.

A joining portion formed by a hook-and-loop fastener or an adhesive can indeed maintain the folded form of the pad 1. However, a hook-and-loop fastener is different from a self-adhesive material in terms that a male fastener exhibits adhesive force not only when brought into contact with a female fastener, but also when brought into contact with other materials, such as nonwoven fabric. Similarly, an adhesive is different from a self-adhesive material in terms that it exhibits adhesive force when brought into contact with materials other than the adhesive. In this case, the joining portion may adhere to the wearer's skin or to clothing or surfaces of the pad 1 other than the joining portion, which may impair easy wearability or cause an unnatural feeling during wear. Particularly, a hook-and-loop fastener is a material having high stiffness and may impair the texture against the wearer's skin if provided on the skin-side surface. Thus, by composing the joining portion 30 from a material other than a hook-and-loop fastener and an adhesive, it is possible to ensure easy wearability and alleviate an unnatural feeling during wear.

Further, it is preferable that the joining portions 30 are disposed on the skin-side surface of the pad 1. With this configuration, when the pad 1 is folded with its skin-side surface-which is for receiving and absorbing excreted fluid-facing inward, the joining portions 30 will contact each other, and the folded state of the pad 1 can be maintained. Thus, excreted fluid and odor therefrom can be contained inside the folded pad 1. Further, exposure of the skin-side surface, which is the surface facing excrement, can be inhibited, and the user's hand is less likely to contact the excrement-facing surface, thereby enabling hygienic discarding. Also for the pad 1 before use, since the pad is maintained in a folded state with its skin-side surface facing inward, the pad can be stored and carried hygienically. Furthermore, since the joining portions 30 are self-adhesive and do not adhere to the wearer's skin, an unnatural feeling is less likely to occur during wear, even when the joining portions 30 are disposed on the skin-side surface.

Note, however, that the above is not a limitation, and the joining portions 30 may be disposed on the non-skin-side surface of the pad 1. Even in this case, a compact state can be maintained, in which the pad 1 is folded with the non-skin-side surface of the pad 1 facing inward. Further, since the self-adhesive joining portions 30 do not adhere to the outer garment (underwear or diaper), it is possible to prevent the outer garment from excessively restricting the conformability of the pad 1 to the wearer's movement.

Further, when the pad 1 is folded at the lengthwise center line CL, it is preferable that the joining portions 30 have a two-layer portion superposed in the thickness direction, as illustrated in FIG. 3B. In the present embodiment, the joining portions 30 which form a pair of joining portions (the one-side joining portions 30a, 30b and the other-side joining portions 30c, 30d) are superposed to form the two-layer portion. In the modified example of FIG. 6, portions of the joining portion 30 are superposed to form the two-layer portion.

With this configuration, when the pad 1 is folded in two in the lengthwise direction, the one-side joining portions 30a, 30b and the other-side joining portions 30c, 30d contact one another to exhibit adhesive force, and thus, the pad 1 can be maintained in a compact, two-folded state. It will suffice if at least a portion of the one-side joining portion 30a, 30b is superposed on the other-side joining portion 30c, 30d when the pad 1 is folded in two. In this way, the joining portions 30 can adhere to each other.

Preferably, when the pad 11 is folded in two, the one-side joining portions 30a, 30b completely overlap respectively with their corresponding other-side joining portions 30c, 30d. To achieve this, it is preferable that the joining portions 30 are arranged symmetrically with respect to the lengthwise center line CL. More specifically, the joining portions 30 are arranged respectively at positions separated from the lengthwise center line CL1 by a predetermined distance L1 in both the one side and the other side in the lengthwise direction. Further, the joining portions 30 are arranged at positions where the one-side joining portions 30a, 30b, as well as the other-side joining portions 30c, 30d, overlap each other in the width direction (in the case of FIG. 4, at positions separated by a predetermined distance W1 from the widthwise center line C).

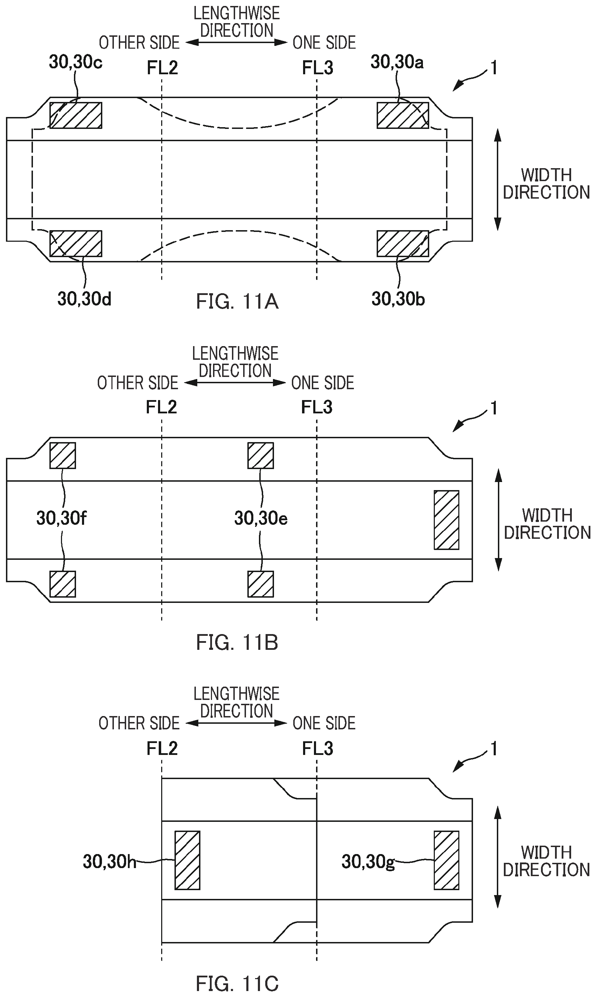

FIGS. 11A to 11C illustrate three-fold-type pads 1. Hereinabove, examples have been described in which the pad 1 is packaged by being folded in two at the lengthwise center line CL serving as the fold line FL1 upon packaging, but this is not a limitation. For example, as illustrated in FIG. 11A, the pad 1 may be packaged by being folded in three at two fold lines FL2, FL3 extending along the width direction. Also in this case, the joining portions 30 may be arranged in the same manner as in the aforementioned pad 1 of FIG. 4 (see FIG. 4). That is, the joining portions 30 may be arranged such that they are superposed on each other when the pad 1 is folded in two at the lengthwise center line CL, and not at the fold lines FL2, FL3 upon packaging. In this way, the user can maintain the pad 1 in a two-folded state and discard the pad 1 in a compact manner. Further, the user only needs to fold the pad once upon discarding, thereby simplifying the discarding task.

The above is not a limitation, and the joining portions 30 may be disposed on the pad 1 such that the pad 1 is maintained in a state folded at the two fold lines FL2, FL3 upon packaging. In this way, the pad 1 can be discarded in a more compact manner. In this case, for example, as illustrated in FIG. 11B, joining portions 30e, 30f may be disposed on the skin-side surface of the pad 1 respectively on one side and the other side in the lengthwise direction orthogonal to the fold line FL2. Further, a joining portion 30g may be disposed on the skin-side surface of the pad 1 on one side in the lengthwise direction relative to the fold line FL3, and a joining portion 30h may be disposed on the non-skin-side surface of the pad 1, which is folded at the fold line FL2, on the other side in the lengthwise direction relative to the fold line FL3. In this way, the pad 1 can be maintained in a state folded in the same manner as when it was packaged.

Further, even in case of a pad 1 that is folded in two at the lengthwise center line CL upon packaging, the joining portions 30 may be arranged such that they contact each other when the pad is folded at a position different from the lengthwise center line CL. Also in this case, the pad 1 can be discarded in a compact manner. Note, however, that in the pad 1 of FIG. 4, a portion of the pad 1 located on one side relative to the fold position and a portion of the pad 1 located on the other side completely overlap. Thus, the inner surface when the pad 1 is folded (in the case of FIG. 4, the skin-side surface which is the excrement-facing surface) is not exposed, thereby enabling the pad to be discarded more hygienically.

Further, as illustrated in FIG. 4, when the pad 1 is divided equally in four in the lengthwise direction, it is preferable that the joining portions 30a, 30b (first joining portions) are disposed in an end region R1 on one side, and the joining portions 30c, 30d (second joining portions) are disposed in an end region R4 on the other side.

With this configuration, outer regions of the pad 1 in the lengthwise direction will be joined together by the joining portions 30. This reduces portions of the pad 1 located outside the joining portions 30 in the lengthwise direction, thereby reducing parts of the pad 1 that would otherwise open up outside the joining portions 30. Thus, the user can readily handle and discard the folded pad 1.

Note, however, that the above is not a limitation, and as in the modified example of FIG. 5, when the pad 1 is divided equally in four in the lengthwise direction, the joining portions 30a, 30b (first joining portions) may be disposed in a first central region R2 (a region immediatley adjacent on one side of the lengthwise center line CL) adjacent to the one-side end region R1, and the joining portions 30c, 30d (second joining portions) may be disposed in a second central region R3 (a region immediatley adjacent on the other side of the lengthwise center line CL) adjacent to the other-side end region R4.

In this case, the joining portions 30 are disposed close to the lengthwise center line CL where the pad 1 is folded in two. Thus, it is easier for the user to align the one-side joining portions 30a, 30b and the other-side joining portions 30c, 30d. Further, the user can stably align the joining portions 30 while gripping the absorbent core 10 which has high stiffness. Thus, the joining portions 30 can be brought into contact reliably, and the fold of the pad 1 can be readily maintained.

It should be noted that, in cases where the absorbent article includes an elastic member as in the present pad 1, the regions R1 to R4-which are defined by dividing the absorbent article in four in the lengthwise direction-are preferably specified with the absorbent article in its unfolded and stretched state. The same applies when specifying or comparing the area, length, position, or the like, as described below.

Further, it is preferable that the length (L30 in FIG. 4) of the joining portion 30 in the lengthwise direction is longer than the length (W30) of the joining portion 30 in the width direction. In cases where the joining portion 30 is composed of a plurality of joining portions 30a to 30d as illustrated in FIG. 4, the length is compared separately for each joining portion 30a to 30d. It is preferable that the aforementioned relationship in length is satisfied in all of the joining portions 30a to 30d.

With this configuration, a wide region of the pad 1 in the lengthwise direction can be joined by the joining portions 30 which are long in the lengthwise direction, thereby making it easy to maintain the folded state of the pad 1. Further, it is possible to reduce the regions of the pad 1 in the lengthwise direction that may open up or separate as a result of not being joined by the joining portions 30. Particularly in cases where the joining portions 30 are disposed on the skin-side surface, excreted fluid and odor therefrom can be contained inside the folded pad 1, thereby enabling the pad 1 to be discarded more hygienically.

Further, it is preferable that the ratio found by dividing the planar area of the joining portion 30 by the planar area of the pad 1 in its unfolded and stretched state (FIG. 4) is 0.15% or higher and below 50%. In cases where the joining portion 30 is composed of a plurality of joining portions 30a to 30d, the total area thereof is referred to as the "planar area of the joining portion 30".

With this configuration, it is easier to bring the joining portions 30 into contact with each other and increase the adhesive strength, compared to cases where the area ratio of the joining portion 30 is below 0.15%. Therefore, it is easier to maintain the pad 1 in its compact folded state. Further, compared to cases where the area ratio of the joining portion 30 is 50% or higher, the pad 1 can be inhibited from being made stiffer by the joining portions 30, thereby ensuring the softness of the pad 1. Particularly in cases where the joining portions 30 are disposed on the skin-side surface, it is possible to prevent the joining portions 30 from covering up the absorption surface of the pad 1 (the region where the absorbent core 10 is arranged) and from reducing the area of the absorption surface.

Further, in the pad 1 of FIG. 4, the joining portions 30 are disposed on the side sheets 4. An example of the side sheet 4 may be a nonwoven fabric subjected to water-repellent treatment. In this way, excreted fluid that has been held back by the leak-proof walls 6 can be inhibited from seeping out. It is preferable that the joining portions 30 are disposed on a non-hydrophilic sheet (water-repellent sheet or hydrophobic sheet), such as the side sheet 4.

It is preferable that the joining portions 30 (the self-adhesive joining agent 32, such as rubber) do not contact moisture. A non-hydrophilic sheet is less likely to absorb and retain excreted fluid compared to a hydrophilic sheet. Therefore, providing the joining portions 30 on a non-hydrophilic sheet makes it less likely for them to contact excreted fluid, thereby inhibiting deterioration in adhesive strength when the joining portions 30 oppose one another.

It is more preferable that, when the pad 1 is folded in two at the lengthwise center line CL, the joining portion 30 does not oppose a hydrophilic sheet. That is, in cases where the joining portion 30 on one side relative to the fold line FL1 opposes a region other than the joining portion 30 on the other side, it is preferable that it opposes a non-hydrophilic sheet, such as the side sheet 4. In this way, the one-side joining portion 30 is less likely to contact excreted fluid, thereby inhibiting deterioration in adhesive strength when the joining portions 30 oppose one another.

Examples of the non-hydrophilic sheet may include nonwoven fabrics and resin films made from non-hydrophilic fibers, such as polyethylene (PE), polypropylene (PP), ethylene-vinyl acetate copolymer (EVA), polyethylene terephthalate (PET), or the like, and sheets made by subjecting a hydrophilic sheet to a water-repellent treatment.

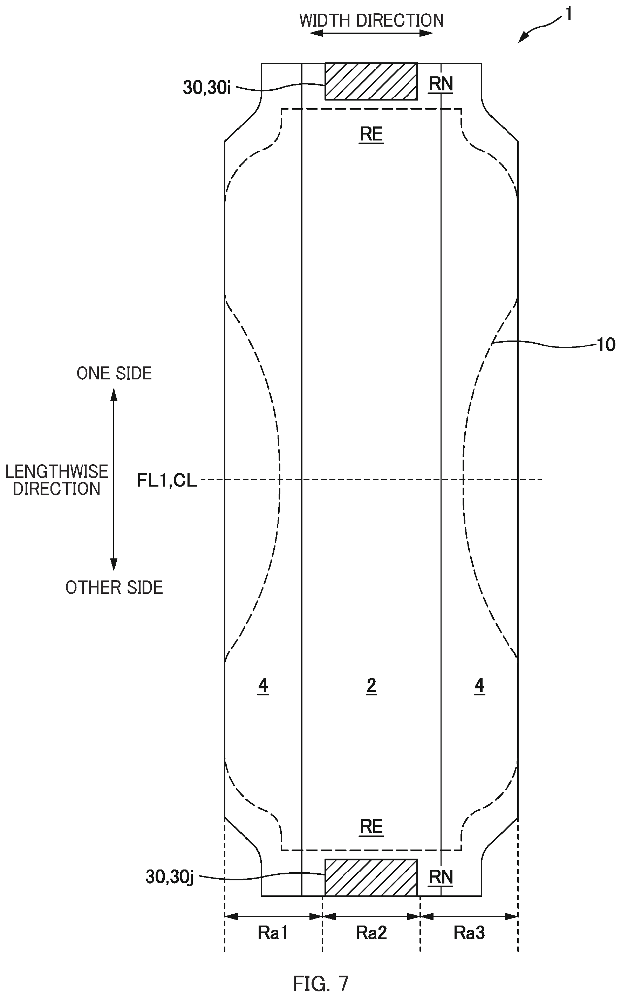

In contrast to the above, the joining portions 30 may be disposed on a hydrophilic sheet, such as the top-surface sheet 2, as in the pad 1 of FIG. 7. A hydrophilic sheet absorbs excreted fluid, but excreted fluid readily diffuses in the planar directions on the hydrophilic sheet, and thus, pooling of excreted fluid is less likely to occur. Therefore, by disposing the joining portions 30 on a hydrophilic sheet, it is possible to avoid the risk that the joining portions 30 will contact a pool of fluid, thereby inhibiting deterioration in joining strength when the joining portions 30 oppose one another.

Examples of the hydrophilic sheet may include nonwoven fabrics made from hydrophilic fibers, such as regenerated cellulose fibers (e.g., rayon), natural fibers (e.g., cotton or pulp), or the like, and sheets made by subjecting a non-hydrophilic nonwoven fabric to a hydrophilizing treatment.

The non-hydrophilic sheet and hydrophilic sheet can be specified by visual observation as to whether or not it is one of the sheets listed above. Alternatively, the sheet can be specified by bringing ion-exchanged water into contact with the surface of the sheet (the surface on which the joining portion 30 is disposed) and measuring the contact angle between the sheet and ion-exchanged water. More specifically, ion-exchanged water (approximately 20 picoliters) is dropped onto the surface of the sheet being measured, and immediately thereafter, the contact angle is measured with a contact angle meter (Contact Angle Meter MCA-J from Kyowa Interface Science Co., Ltd., or an equivalent thereof). The measurement is performed at a plurality of sites (e.g., five or more sites) on the surface of the sheet, and the average value thereof is found as the contact angle. The measurement environment temperature is 22°C. If the contact angle is less than 90 degrees, the sheet is specified as a hydrophilic sheet, and if the contact angle is 90 degrees or greater, the sheet is specified as a hydrophobic sheet.

Further, in cases where the joining portion 30 is disposed on the skin-side surface of the pad 1, it is preferable that the mean deviation of surface roughness (SMD) on the joining face (skin-side surface) of the joining portion 30 is 20 or less. In this way, the roughness and uneven feeling on the surface of the joining portion 30 can be reduced and the texture of the joining portion 30 against the skin can be improved, compared to cases where the mean deviation of surface roughness (SMD) is greater than 20. Typically, the SMD of a male member of a hook-and-loop fastener is greater than 20, so by using the aforementioned self-adhesive joining portion 30, the burden on the wearer's skin can be reduced and an unnatural feeling during wear can be alleviated, compared to a male member of a hook-and-loop fastener.

Further, it is preferable that the mean frictional coefficient (MIU) on the joining face of the joining portion 30 is 0.2 or greater. In this way, slipperiness of the joining portion 30 can be reduced, compared to cases where the mean frictional coefficient (MIU) is less than 0.2. Therefore, it is possible to inhibit misalignment between the wearer and the joining portion 30 disposed on the skin-side surface of the pad 1 and misalignment between an outer garment (underpants-shaped diaper or underwear) and the joining portion 30 disposed on the non-skin-side surface of the pad 1. Thus, it is possible to alleviate an unnatural feeling during wear.

The mean deviation of surface roughness (SMD) and the mean frictional coefficient (MIU) can be measured according to the following method. First, the joining portion 30 is cut out from the pad 1 to be measured, to prepare a sample piece. Next, using an automatic surface tester KES-FB4-AUTO-A from Kato Tech Co., Ltd., the sample piece is set onto a test stage with a flat metal surface. A probe applied with a load of 25 gf is slid on the surface of the test piece (the joining face of the joining portion 30) at a speed of 1.0 mm/second, to measure the mean deviation of surface roughness (SMD) and the mean frictional coefficient (MIU). The probe has a size of 10×10 mm, and a 0.5 mm-diameter piano wire is wound thereon. It is preferable that a plurality of test pieces are prepared, and the average of measurement values for the test pieces is found as the mean deviation of surface roughness (SMD) and the mean frictional coefficient (MIU).

Further, it is preferable that, when an "adhesive force measurement test" is performed to measure the adhesive force between the joining portions 30 after repeatedly joining (adhering) and peeling the joining portions 30, the adhesive force between the joining portions 30 is 0.2 N/25 mm or greater.

In this way, compared to cases where the adhesive force after the test is less than 0.2 N/25 mm, adhesive force when the joining portions 30 are in contact can be secured, even after repeatedly joining and peeling the joining portions 30 a plurality of times (three times). Therefore, adhesive force can be secured, even when a user repeatedly joins and peels the joining portions 30 to perform alignment until the one-side joining portion 30 and the other-side joining portion 30 are brought into contact appropriately while folding the used pad 1. Thus, the pad 1 can be discarded while maintaining its compact folded state.

The adhesive force measurement test can be performed using a tensile tester (Model 5965 from Instron, or an equivalent thereof). First, from the pad 1 to be measured, a joining portion 30 on one side relative to the fold line FL1 and a joining portion 30 on the other side are cut out, to prepare sample pieces. Each sample piece has a rectangular shape in which the lengthwise direction is the peeling direction (tensile direction), wherein, for example, the sample piece's length in the peeling direction and the width is 40 mm and 25 mm, respectively. However, to constitute the entire region of the sample piece by the joining portion 30, the size of the sample piece is adjusted in accordance with the size of the joining portion 30.

Next, the sample piece composed of the one-side joining portion 30 and the sample piece composed of the other-side joining portion 30 are joined and peeled repeatedly three times. Then, in a state where the sample piece composed of the one-side joining portion 30 and the sample piece composed of the other-side joining portion 30 are joined together, the respective ends of the test pieces in the peeling direction are pinched with respective chucks of the tensile tester (chuck-to-chuck distance: e.g., 20 mm). Then, the chuck-to-chuck distance is widened at a tensile speed of 100 mm/minute until the sample piece composed of the one-side joining portion 30 and the sample piece composed of the other-side joining portion 30 are peeled apart. The maximum value of the measurement values (tensile loads) measured during this period is found as the adhesive force (N/25 mm) between the joining portions 30.

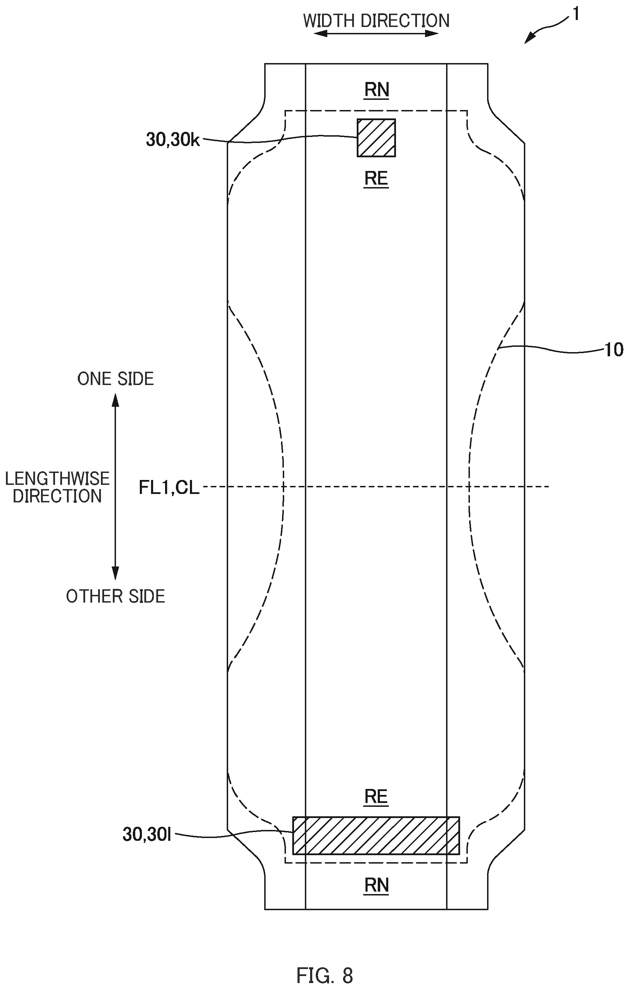

In the pad 1 illustrated in FIG. 4, the four joining portions 30a to 30d constituting the joining portion 30 all have the same shape and area. This, however, is not a limitation, and as in the modified example illustrated in FIG. 8, the area of the joining portion 30k (first joining portion) disposed on one side relative to the fold line FL1 may be different from the area of the joining portion 30l (second joining portion) disposed on the other side relative to the fold line FL1. For example, it is preferable that the area of the larger joining portion 30 is greater than or equal to 110% of the area of the smaller joining portion 30.

With this configuration, the user can easily align the smaller joining portion (in this example, the joining portion 30k) with respect to the larger joining portion (in this example, the joining portion 30l) and easily bring the joining portions 30 into contact with each other. Further, in cases where the one-side joining portion 30 and the other-side joining portion 30 have the same area and shape, the user may feel a sense of burden in aligning the joining portions 30 exactly. With the aforementioned configuration, however, such a risk can be eliminated. Note, however, that when the one-side joining portion 30 and the other-side joining portion 30 have the same area and shape as illustrated in FIG. 4, the adhesive force can be increased when the joining portions 30 are aligned with minimal deviation, which enables the joining portions 30 to be employed effectively.

Further, the planar-view shape of the absorbent core 10 is smaller than the planar-view shape of the pad 1. Thus, the pad 1 includes a presence region RE in which the absorbent core 10 is present, and an absence region RN in which the absorbent core 10 is not present.

In this case, as in the pad 1 illustrated in FIG. 8, it is preferable that the joining portion 30 is disposed in the presence region RE in which the absorbent core 10 is present. By positioning the joining portion 30 on the absorbent core 10 having high stiffness, the force applied by the user to bring the joining portions 30 into contact can be readily transmitted. Further, the user can bring the joining portions 30 into contact stably while gripping a highly-stiff region where the absorbent core 11 is present. Thus, the contact surface between the joining portions 30 can reliably exhibit adhesive force and can be joined together securely, thereby readily maintaining the pad 1 in a compact state.

The above is not a limitation, and the joining portions 30 may be disposed in an absence region RN where the absorbent core 10 is not present, as in the pad 1 of FIG. 7.

The absence region RN is located in a peripheral edge portion of the pad 1. Therefore, in the above case, the joining portions 30 can be disposed in the peripheral edge portion of the pad 1. This makes it easy to maintain the pad 1 in a folded state. Further, it is possible to reduce parts of the pad 1 that would otherwise open up outside the joining portions 30 in the lengthwise direction or the width direction. Particularly in cases where the joining portions 30 are disposed on the skin-side surface of the pad 1, by keeping the joining portions 30 from covering up the absorption surface of the pad 1 (the region where the absorbent core 10 is arranged), the area of the absorption surface for receiving and absorbing excreted fluid can be secured.

Alternatively, as in the pad 1 of FIG. 4 or FIG. 5, the joining portion 30 may be disposed extending across the presence region RE and the absence region RN.

With this configuration, the joining portions 30 can be brought into contact stably in the highly-stiff presence region RE and can be joined together securely, while also disposing the joining portions 30 in the peripheral edge portion of the pad 1. Further, in cases where the joining portion 30 is disposed on the skin-side surface of the pad 1, it is possible to reliably secure the area of the absorption surface, compared to cases where the entire region of the joining portion 30 is located in the presence region RE.

Further, although not illustrated, one of the joining portion 30 (i.e., the first joining portion) on one side relative to the fold line FL1 and the joining portion 30 (i.e., the second joining portion) on the other side may be disposed in the presence region RE, and the other may be disposed in the absence region RN.

With this configuration, one of the joining portions 30 will be disposed in the peripheral edge and can inhibit regions of the pad 1 outside the joining portions 30 from opening up. Further, the one-side joining portion 30 can stably be brought into contact with the other joining portion 30 located in the highly-stiff presence region RE, thereby allowing the joining portions 30 to be joined together securely. Further, in cases where the joining portions 30 are disposed on the skin-side surface of the pad 1, it is possible to reliably secure the area of the absorption surface, compared to cases where both the one-side and other-side joining portions 30 are located in the presence region RE.

Further, it is preferable that the joining portion 30 includes a plurality of sets of joining portions 30 (first joining portions) disposed on one side relative to the fold line FL1 and joining portions (second joining portions) disposed on the other side relative to the fold line FL1. For example, the pad 1 of FIG. 4 includes two sets, i.e., a set of the one-side joining portion 30a and the other-side joining portion 30c corresponding thereto, and a set of the one-side joining portion 30b and the other-side joining portion 30d corresponding thereto. That is, the joining portions 30 are disposed intermittently in the width direction. Although not illustrated, the joining portions 30 may be disposed intermittently in the lengthwise direction on each of the one side and the other side relative to the fold line FL1 to thereby provide a plurality of sets of one-side joining portions 30 and other-side joining portions 30.

By providing a plurality of sets of the joining portions 30 which contact each other when the pad 1 is folded, the pad 1 becomes less likely to open up, thereby maintaining its compact state more easily. Further, by providing the joining portions 30 intermittently, the stiffness of the pad 1 can be reduced, the amount of material can be reduced, and an unnatural feeling during wear can be alleviated, compared to cases where the joining portions 30 are provided continuously in the lengthwise direction or the width direction.

Further, as in the pad 1 of FIG. 4, it is preferable that the joining portion 30 is disposed in a widthwise end region Ra1, Ra3 when the pad 1 is divided equally in three in the width direction.

With this configuration, the user can bring the one-side joining portion 30 and the other-side joining portion 30 into contact while folding the pad 1 by aligning the end portions thereof, thereby facilitating contact between the joining portions 30.

In this case, as in the pad 1 of FIG. 4, it is preferable that the joining portion 30 is disposed not only in the one-side end region Ra1 in the width direction, but also in the other-side end region Ra3 in the width direction. That is, it is preferable that two sets of joining portions-each composed of the one-side joining portion 30 and the other-side joining portion 30 relative to the fold line FL1-are provided, and the two sets of joining portions 30 are disposed respectively at both end portions of the pad 1 in the width direction. With this configuration, the four corners of the pad 1 can be joined, thereby further inhibiting the pad 1 from opening up and enabling the pad to easily maintain its compact state.

The above is not a limitation, and as in the pad 1 of FIG. 7, the joining portions 30 may be disposed in the widthwise central region Ra2 when the pad is divided equally in three in the width direction.

In this case, opening of both lateral sides of the pad 1 in the width direction can be inhibited in a balanced manner while reducing the number of joining portions 30. By reducing the number of sets of joining portions 30 on the one side and the other side relative to the fold line FL1, it is possible to alleviate the user's sense of burden in aligning the plurality of sets of joining portions 30 exactly.

### SECOND EMBODIMENT:

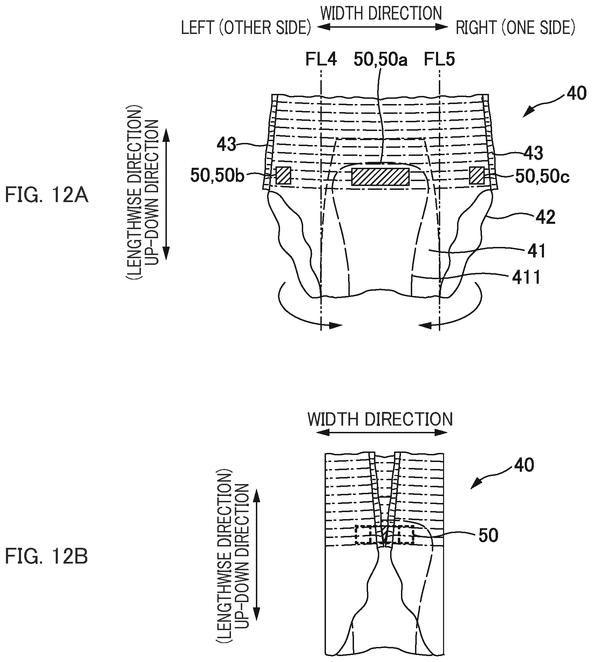

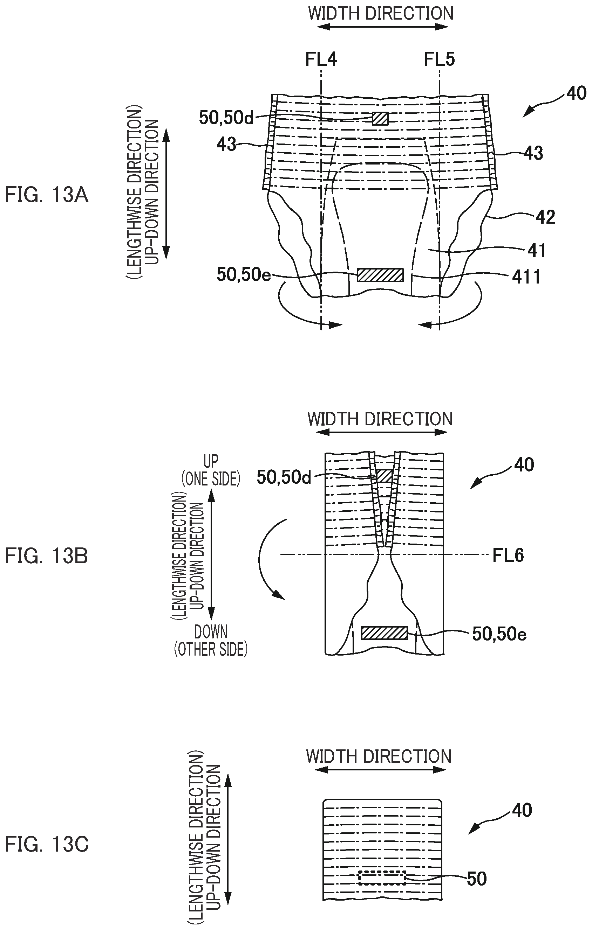

In a second embodiment, an underpants-shaped diaper 40 (also referred to hereinafter as "diaper 40") will be described as an example of an absorbent article. FIGS. 12 and 13 are diagrams illustrating self-adhesive joining portions 50 in a diaper 40.

The diaper 40 includes an absorbent main body 41 and an exterior member 42. The absorbent main body 41 includes an absorbent core 411. The diaper 40 in an underpants-shaped state has an up-down direction and a width direction orthogonal to each other, and the diaper 40 in an unfolded state (not illustrated) has a lengthwise direction, a width direction, and a thickness direction orthogonal to one another. The lengthwise direction of the diaper 40 corresponds to the up-down direction of the diaper 40 in its underpants-shaped state and is a direction along the lengthwise direction of the absorbent main body 41. In the exterior member 42, a front waist portion which contacts the wearer's front portion and a back waist portion which contacts the wearer's back portion are mutually joined and connected by side joining portions 43 at both side portions in the width direction. In this way, a waist opening and a pair of leg openings are formed, to form an underpants shape.

The diaper 40 (particularly, an adult-use diaper 40 with a large size) may be packaged in a state in which portions of the exterior member 42 which extend outward from the absorbent main body 41 in the width direction are folded at fold lines FL4, FL5, as illustrated in FIGS. 12A and 12B. Further, the diaper 40 may be packaged in a state folded in the up-down direction (lengthwise direction) at fold line FL6, as illustrated in FIGS. 13B and 13C.

It is preferable that the diaper 40 also includes self-adhesive joining portions 50, as in the pad 1 of the first embodiment. Further, it is preferable that the joining portions 50 are disposed on one side and the other side relative to each fold line FL4 to FL6 in a direction orthogonal to each fold line FL4 to FL6 upon packaging. With this configuration, the user can bring the one-side joining portion 50 and the other-side joining portion 50 into contact while folding the diaper 40, and the diaper 40 can thereby maintain a compact folded state. Further, since the joining portions 50 are self-adhesive, they do not adhere to items such as clothing when putting on the diaper 40, thereby ensuring easy wearability.

More specifically, as illustrated in FIG. 12A, it is preferable to dispose joining portion 50a on the right side (one side) relative to the fold line FL4 upon packaging and dispose joining portion 50b on the left side (other side) in the width direction orthogonal to the fold line FL4 upon packaging. Further, it is preferable to dispose joining portion 50b on the right side (one side) relative to the fold line FL5 and dispose, on the other side, joining portion 50a on the left side (other side) in the width direction. With this configuration, as illustrated in FIG. 12B, the user can bring the central joining portion 50a and the side joining portions 50b, 50c into contact while folding both side portions, in the width direction, of the exterior member 42 inwardly in the width direction, thereby enabling the joining portions 50 to exhibit adhesive force. Thus, the user can carry or discard the diaper 40 which is maintained in a compact state in the width direction.

It should be noted that the joining portion 50 on the one side relative to the fold line FL4 and the joining portion 50 on the other side relative to the fold line FL5 may be a continuous common joining portion as illustrated in FIG. 12A, or may be two separate joining portions.

Further, as illustrated in FIG. 13B, joining portion 50d may be disposed on the upper side (one side) relative to the fold line FL6 and joining portion 50e may be disposed on the lower side (other side) in the up-down direction (lengthwise direction) orthogonal to the fold line FL6 upon packaging. With this configuration, as illustrated in FIG. 13C, the user can bring the joining portion 50d and the joining portion 50e into contact while folding the diaper 40 in the up-down direction, thereby enabling the joining portions 50 to exhibit adhesive force. Thus, the user can carry or discard the diaper 40 which is maintained in a compact state in the up-down direction.

The aforementioned fold lines FL4 to FL6 upon packaging and joining portions 50 are merely examples and are not limited thereto. For example, the diaper 40 may include only the fold line FL6 along the width direction, or may include all of the joining portions 50a to 50c of FIG. 12A and the joining portions 50d, 50e of FIG. 13B.

Further, the diaper 40 includes a presence region in which the absorbent core 411 is present, and an absence region in which the absorbent core 411 is not present. In the diaper 40 of FIG. 12A, one of the joining portions (the joining portion 50a), among the one-side joining portion 50a and the other-side joining portion 50b relative to the fold line FL4, is disposed in the presence region, and the other (the joining portion 50b) is disposed in the absence region. Similarly, one of the joining portions (the joining portion 50a), among the one-side joining portion 50c and the other-side joining portion 50a relative to the fold line FL5, is disposed in the presence region, and the other (the joining portion 50c) is disposed in the absence region.

In this case, the joining portions 50b, 50c-which are located on the exterior member 42 having low stiffness and being easy to fold since the absorbent core 411 is not present-can be brought closer to the joining portion 50a having high stiffness, and can be brought into contact stably. Thus, the joining portions 50 can be joined together securely.

### OTHERS:

The foregoing embodiments facilitate understanding of the present invention and are not to be construed as limiting the present invention. It goes without saying that the present invention may variously be changed or altered without departing from its gist and encompasses equivalents thereof.

### REFERENCE SIGNS LIST

1: Urine collection pad (absorbent article);
2: Top-surface sheet;
3: Back-surface sheet;
4: Side sheet;
5: Leg elastic member;
6: Leak-proof wall portion;
7: Leak-proof-wall elastic member;
10: Absorbent core;
11: Core-wrapping sheet;
20: Fixing member;
30: Joining portion;
30a: Joining portion (first joining portion);
30b: Joining portion (first joining portion);
30c: Joining portion (second joining portion);
30d: Joining portion (second joining portion);
40: Underpants-shaped diaper (absorbent article);
41: Absorbent main body;
411: Absorbent core;
42: Exterior member;
43: Side joining portion;
50: Joining portion.

## Claims

1. An absorbent article having, in an unfolded state, a lengthwise direction, a width direction, and a thickness direction orthogonal to one another, the absorbent article comprising:
a liquid-absorbent absorbent core; and
a self-adhesive joining portion,
the joining portion being disposed, in a direction orthogonal to a fold line upon packaging, on one side relative to the fold line and also on an other side relative to the fold line.

2. The absorbent article according to claim 1, wherein
when the absorbent article is folded at a center line of the absorbent article in the lengthwise direction, the joining portion includes a two-layer portion superposed in the thickness direction.

3. The absorbent article according to claim 2, wherein
the joining portion includes a first joining portion disposed on the one side relative to the fold line and a second joining portion disposed on the other side relative to the fold line, and
when the absorbent article is divided in four in the lengthwise direction, the first joining portion is disposed in an end region on one side, and the second joining portion is disposed in an end region on an other side.

4. The absorbent article according to claim 2, wherein
the joining portion includes a first joining portion disposed on the one side relative to the fold line and a second joining portion disposed on the other side relative to the fold line, and
when the absorbent article is divided in four in the lengthwise direction, the first joining portion is disposed in a first central region adjacent to an end region on one side, and the second joining portion is disposed in a second central region adjacent to an end region on an other side.

5. The absorbent article according to any one of claims 1 to 4, wherein
the joining portion is disposed on a skin-side surface of the absorbent article.

6. The absorbent article according to any one of claims 1 to 4, wherein
a length of the joining portion in the lengthwise direction is longer than a length of the joining portion in the width direction.

7. The absorbent article according to any one of claims 1 to 4, wherein
the joining portion is disposed on a non-hydrophilic sheet.

8. The absorbent article according to any one of claims 1 to 4, wherein
the joining portion is disposed on a hydrophilic sheet.

9. The absorbent article according to any one of claims 1 to 4, wherein
the joining portion exhibits adhesive force by a material other than a hook-and-loop fastener and an adhesive.

10. The absorbent article according to claim 5, wherein
a mean deviation of surface roughness on a joining face of the joining portion is 20 or less.

11. The absorbent article according to any one of claims 1 to 4, wherein
a mean frictional coefficient on a joining face of the joining portion is 0.2 or greater.

12. The absorbent article according to any one of claims 1 to 4, wherein
when an adhesive force measurement test is performed to measure an adhesive force between the joining portion after repeatedly joining and peeling the joining portion, the adhesive force between the joining portion is 0.2 N/25 mm or greater.

13. The absorbent article according to any one of claims 1 to 4, wherein
the joining portion includes a first joining portion disposed on the one side relative to the fold line and a second joining portion disposed on the other side relative to the fold line, and
an area of the first joining portion is different from an area of the second joining portion.

14. The absorbent article according to any one of claims 1 to 4, wherein
the absorbent article comprises a presence region in which the absorbent core is present, and an absence region in which the absorbent core is not present, and
the joining portion is disposed in the presence region.

15. The absorbent article according to any one of claims 1 to 4, wherein
the absorbent article comprises a presence region in which the absorbent core is present, and an absence region in which the absorbent core is not present, and
the joining portion is disposed in the absence region.

16. The absorbent article according to any one of claims 1 to 4, wherein
the absorbent article comprises a presence region in which the absorbent core is present, and an absence region in which the absorbent core is not present, and
the joining portion is disposed extending across the presence region and the absence region.

17. The absorbent article according to any one of claims 1 to 4, wherein
the joining portion includes a first joining portion disposed on the one side relative to the fold line and a second joining portion disposed on the other side relative to the fold line,
the absorbent article comprises a presence region in which the absorbent core is present, and an absence region in which the absorbent core is not present, and
one of the first joining portion and the second joining portion is disposed in the presence region, and the other is disposed in the absence region.

18. The absorbent article according to any one of claims 1 to 4, wherein
the joining portion is disposed in a widthwise central region when the absorbent article is divided in three in the width direction.

19. The absorbent article according to any one of claims 1 to 4, wherein
the joining portion is disposed in a widthwise end region when the absorbent article is divided in three in the width direction.

20. The absorbent article according to any one of claims 1 to 4, wherein
the joining portion includes a plurality of sets of a first joining portion disposed on the one side relative to the fold line and a second joining portion disposed on the other side relative to the fold line.
